# FASCICULE DE BREVET EUROPEEN

(11) **EP 1 239 836 B1**
(45) Date de publication et mention de la délivrance du brevet: **20.08.2003**
(21) Numéro de dépôt: 00968011.7
(22) Date de dépôt: 11.10.2000
(51) Int. Cl.: A61K 9/16, A61K 9/51, B01J 13/00

(54) **SUSPENSION COLLOIDALE DE PARTICULES SUBMICRONIQUES DE VECTORISATION DE PRINCIPES ACTIFS ET LEUR MODE DE PREPARATION**
KOLLOIDALE SUSPENSION VON SUBMIKRONISCHEN TEILCHEN ALS VEKTOREN VON AKTIVEN PRINZIPIEN SOWIE DEREN HERSTELLUNG
COLLOIDAL SUSPENSION OF SUBMICRONIC PARTICLES AS VECTORS FOR ACTIVE PRINCIPLES AND METHOD FOR PREPARING SAME

(30) Priorité: 23.11.1999 FR 9914751
(43) Date de publication de la demande: 18.09.2002
(73) Titulaire: FLAMEL TECHNOLOGIES, 69693 Venissieux Cédex (FR)
(72) Inventeur: TOURAUD, Franck, F-69003 LYON (FR); BRYSON, Nathan, F-69390 Millery (FR)
(74) Mandataire: Fleurance, Raphael
(86) Numéro de dépôt international: FR0002831
(87) Numéro de publication internationale: WO01037809

(56) Documents cités:
- FR-A- 2 746 035
- US-A- 5 904 936

## Description

### DOMAINE TECHNIQUE

Le domaine de la présente invention est celui des Particules de Vectorisation (**PV**), utiles pour l'administration de principes actifs (**PA**). Ces derniers sont, de préférence, des médicaments ou des nutriments pour l'administration à un organisme animal ou humain par voie orale ou nasale, vaginale, oculairc, sous-cutanée, intraveineuse, intramusculaire, intradermique, intrapéritonéale, intracérébrale, parentérale, etc... Mais il peut s'agir aussi de produits cosmétiques ou de produits phytosanitaires, tels que des herbicides, des pesticides, des insecticides, des fongicides, etc. En terme de nature chimique, les **PA** plus particulièrement, mais non limitativement, concernés par l'invention sont, par exemple, des protéines, des glycoprotéines, des peptides, des polysaccharides, des lipopolysaccharides, des oligonucléotides, des polynucléides et des molécules organiques.

La présente invention concerne, plus précisément, des suspensions colloïdales de Particules de Vectorisation, avantageusement de type submicronique, à base de polyaminoacides (PAA). La présente invention vise aussi bien des particules nues en tant que telles, que les systèmes de vecteurs de **PA,** constitués par les particules chargées par le (ou les) **PA** considéré(s). La présente invention a également trait à des solides pulvérulents comprenant ces **PV.** L'invention concerne, également, des procédés de préparation desdites suspensions colloïdales de particules, avec ou sans **PA.**

### ART ANTERIEUR

L'encapsulation de **PA** dans les **PV** a notamment, pour but de modifier leur durée d'action et/ou de les acheminer au lieu du traitement et/ou augmenter la biodisponibilité desdits **PA.** De nombreuses techniques d'encapsulation ont déjà été proposées. De telles techniques visent, d'une part, à permettre le transport du **PA** jusqu'à son site d'action thérapeutique, tout en le protégeant contre les agressions de l'organisme (hydrolyse, digestion enzymatique, etc.) et, d'autre part, à contrôler la libération du **PA** sur son site d'action, afin de maintenir la quantité disponible pour l'organisme au niveau désiré. Les **PA** concernés par ces avatars de transport et de séjour dans l'organisme sont, par exemple, des protéines mais peuvent être, également, des produits tout autres, des molécules organiques d'origine synthétique ou naturelle.

La revue de M.J. HUMPHREY (Delivery system for peptide Drugs, éditée par S. DAVIS et L.ILLUM, Plenum Press, N.Y. 1986), fait état de la problématique concernant l'amélioration de la biodisponibilité des PA et l'intérêt des systèmes de vectorisation et de libération contrôlée.

Parmi tous les matériaux envisageables pour former des **PV ,** les polymères sont de plus en plus utilisés, du fait de leurs propriétés intrinsèques. S'agissant du cahier des charges que l'on souhaite obtenir pour les PV, il est particulièrement exigeant et comprend, notamment, les spécifications suivantes .
1 La première spécification recherchée pour les **PV** serait que le polymère, constituant les **PV** soit biocompatible, éliminable (par excrétion) et/ou biodégradable et, encore mieux, qu'il soit métabolisé en produits non toxiques pour l'organisme. En outre, il conviendrait que la biodégradation dans l'organisme soit d'une durée suffisamment courte.
2 Les **PV** auraient avantage à pouvoir former, sans l'aide de solvant organique et/ou de tensioactif, une suspension aqueuse stable.
3 Il serait également souhaitable que les **PV** aient une taille suffisamment faible pour pouvoir subir, en suspension dans un liquide, une filtration stérilisante par un filtre dont le diamètre des pores est inférieur ou égal à 0,2 µm.
4 Il est souhaitable que les **PV** et les systèmes **PV-PA** puissent être obtenus par un procédé non dénaturant pour le **PA**.
5 Les **PV** devraient, avantageusement, permettre de contrôler la vitesse de libération du **PA**.
6 Une autre spécification importante serait que les systèmes **PV-PA** puissent constituer d'excellents médicaments injectables. Cette aptitude améliorée de l'administration par injection -e.g. intraveineuse ou intramusculaire- « injectabilité» se caractérise par :
   (i) un volume injecté réduit (pour une dose thérapeutique donnée)
   (ii) une viscosité faible.
   Ces deux propriétés sont satisfaites lorsque la dose thérapeutique de **PA** est associée à une quantité minimale de **PV.** En d'autres termes, les **PV** doivent avoir un fort taux de chargement en **PA.**
7 Le coût propre aux **PV** dans une préparation injectable doit être réduit et là encore il convient que les **PV** aient un fort taux de chargement en **PA.** En définitive, la fiable taille et un fort taux de chargement sont des spécifications majeures recherchées pour les **PV.**
8 Il est également avantageux que le polymère, constitutif des **PV,** n'induise pas de réponse immunitaire.

Les propositions techniques antérieures, décrites infra, ont tenté de satisfaire l'ensemble de ces spécifications. A titre d'illustration, on citera les propositions antérieures (a) à (h) :
(a) Le brevet US-A-5 286 495 concerne un procédé d'encapsulation par vaporisation de protéines en phase aqueuse, à l'aide de matériaux ayant des charges opposées, à savoir : l'alginate (chargé négativement) et la polylysine (chargée positivement). Ce procédé de fabrication permet de produire des particules de taille supérierue à 35 µm.
(b) Par ailleurs, les techniques d'émulsion sont couramment utilisées pour préparer des microparticules chargées de **PA** .Par exemple, les demandes de brevets WO 91/06286, WO 91/06287 et WO 89/08449 divulguent de telles techniques d'émulsion dans lesquelles on a recours à des solvants organiques pour solubiliser des polymères, par exemple de type polylactique. Mais il s'est avéré que les solvants peuvent être dénaturants, notamment pour les **PA** peptidiques ou polypeptidiques.
(c) On connaît, également, des **PV** biocompatibles appelées protéinoïdes, décrites dès 1970 par X. FOX et K. DOSE dans « Molecular Evolution and the origin of Life », Ed. Marcel DEKKER Inc (1977). Ainsi, la demande de brevet WO 88/01213 propose un système à base d'un mélange de polypeptides synthétiques, dont la solubilité dépend du pH. Pour obtenir les microparticules matricielles selon cette invention, ils solubilisent le mélange de polypeptides, puis avec un changement de pH, ils provoquent la précipitation de particules protéinoïdes. Lorsque la précipitation s'effectue en présence d'un **PA,** celui-ci est encapsulé dans la particule.
(d) On mentionnera également, pour mémoire, le brevet US 4 351 337 qui relève d'un domaine différent de celui de la vectorisation de **PA** propre à l'invention. Ce brevet divulgue des implants massiques fixés et localisés à des endroits bien précis de l'organisme. Ces implants sont des tubes ou des capsules creuses de taille microscopiques (160 µm et de longueur égale à 2 000 µm), constitués de copolymères de copoly(aminoacides) - e.g. poly(acide glutamique-leucine) ou poly(benzylglutamate-leucine) - obtenus par copolymérisation de monomères de N-carboxyanhydrides d'aminoacides (NCA). L'inclusion d'un **PA** s'opère par une technique d'évaporation de solvant d'un mélange de polymère et de PA. Le brevet US 4 450 150 appartient à la même famille que le brevet US 4 351 337 étudié ci-dessus et a essentiellement le même objet. Les PAA constitutifs sont des poly(acide glutamique-éthylglutamate).
(e) La demande de brevet PCT/FR WO 97/02810 divulgue une composition pour la libération contrôlée de principes actifs, comprenant une pluralité de particules lamellaires d'un polymère biodégradable, au moins en partie cristallin (polymère d'acide lactique) et d'un **PA** absorbé sur lesdites particules. Dans ce cas, la libération du principe actif s'opère par désorption.
(f) La publication « CHEMISTRY LETTERS 1995, 707, AKIYOSHI ET AL » concerne la stabilisation d'insuline par complexation supramoléculaire avec des polysaccharides hydrophobisés par greffage de cholestérol.
(g) L'article paru dans «MACROMOLECULES 1997, 30, 4013-4017 » décrit des copolymères composés d'un bloc polypeptide à base de L-phénylalanine, de (-benzyl-L-glutamate ou de O-(tétra-O-acétl-D-glucopyranosyl)-L-sérine, et un bloc synthétique, tels que la poly(2-méthyl-2-oxazoline) ou la poly(2-phényl-2-oxazoline). Des polymères s'agrègent en milieu aqueux pour former des particules de 400 nm, capables de s'associer avec une enzyme, la lipase. La terme associée signifie ici que la protéine s'adsorbe sur la particule par un phénomène physique (pas de liaison covalente).
(h) La demande de brevet FR 2 746 035 décrit notamment page 28 lignes 3 à 16, une suspension colloïdale de microparticules gel composite obtenue à partir d'un polyaminoacide du type polypolyleucine/glutamate de sodium, huile de coco fractionnée (miglyol®) et d'eau désionisée ou de solution saline tamponnée (tampon phosphate pH 7,4 à 25°C). Le diamètre moyen de référence D[4,3] de ces microparticules gel composite est de 2800 nm.
   Il ressort de tous les exemples du FR 2 746 035, que le plus petit diamètre moyen de référence D[4,3] est égal à 1900 nm.
   Par ailleurs, ces microparticules gel composite ne peuvent pas s'associer à l'insuline à l'état non dissous en suspension colloïdale, selon un taux Ta ≥ 7%. Dans ces conditions, il est manifeste que les microparticules gel composite ne satisfont pas au cahier des charges, et notamment pas aux spécifications relatives à l'injectabilité et à la capacité d'association et de libération vis-à-vis de l'insuline.
   En outre, le procédé selon le FR 2 746 035 ne fait pas intervenir de solvant polaire non aromatique et la formation des microparticules n'intervient pas de manière spontanée en milieu aqueux, mais passe par la mise en oeuvre d'une homogénéisation vigoureuse à l'aide d'un dispositif du type rotoristator.
(i) La demande PCT WO 96/29991 a pour objet des particules de polyaminoacides utiles pour la vectorisation de **PA.** Ces particules ont une taille comprise entre 10 et 500 nm, de préférence entre 30 et 400 nm. Dans les exemples de cette demande PCT, la taille des particules est mesurée par le rayon de giration. Le rayon de giration des particules obtenues dans ces exemples varie de 55 à 280 nm. Il existe d'autres techniques de mesure de la taille de particules colloïdales. La détermination du diamètre hydrodynamique moyen (Dh) des particules par diffusion quasi élastique de la lumière (QELS) est un exemple de méthode commode de mesure. Dans tout le présent exposé, on prendra pour référence un mode opératoire Md de mesure de Dh.. Md est décrit plus loin. Ainsi, le Dh des particules selon les exemples du PCT WO 96/29991 s'étend de 150 nm à 750 nm. Il est à remarquer que les **PV** dont il est question ici sont formées d'un coeur hydrophobe entouré d'une chevelure hydrophile. Le diamètre hydrodynamique de ces objets est inférieur au double de leur rayon de gyration, comme cela est expliqué par exemple dans les ouvrages "Dynamic Light Scattering", B.J. Berue and R. Pecaran (Wiley, 1976) and "Physicochemical Hydrodynamics", R.F Probstein (Wiley 1994). Le taux de chargement Ta des particules s'exprime commodément par le rapport de la masse d'insuline à la masse de **PV** sec. Selon les exemples du WO 96/29991, avec un **PA** constitué par de l'insuline, est au mieux de 0,065 mg/mg, soit 6,5 % en poids sec d'insuline par rapport à la masse de PAA. Ta est mesuré selon un mode opératoire Ma décrit plus loin. Les particules selon le WO 96/29991 se forment spontanément par mise en contact de PAA avec une solution aqueuse. Les PAA comprennent des monomères aminoacides neutres et hydrophobes AAO et des monomères ionisables et hydrophiles AAI. Ces PAA sont préparés par copolymérisation de NCA de précurseurs d'AAI (e.g. : Glu-OMe) et de NCA d'AAO (e.g. Leu) en solution dans un mélange dioxane/toluène. Le copoly(Glu-OMe)(Leu) obtenu en solution est récupéré par précipitation dans l'eau, filtration et séchage. Ce copolymère est alors soumis à une hydrolyse acide en l'incorporant dans l'acide TriFluoroAcétique (TFA), dans lequel il se dissous. Un copolymère (Glu-O-Na)(Leu) est récupéré après neutralisation, dialyse, filtration et lyophilisation. Ce coPAA est dispersé dans une solution aqueuse de NaCl et il se forme spontanément une suspension de nanoparticules. Comme indiqué supra, ces dernières sont de taille Dh supérieure à 150 nm et un taux de chargement à l'insuline Ta 6.50%.

Il ressort donc de ce qui précède que les propositions techniques antérieures sus décrites, et notamment la proposition (i), satisfont incomplètement aux spécifications du nouveau cahier des charges indiqué supra, et, en particulier une aptitude à la stérilisation par filtration, une haute vitesse de dégradation, une adaptabilité aux contraintes de l'administration de médicaments par injection, un faible coût et un fort taux de chargement en **PA.**

S'agissant de l'aptitude à la filtration stérilisante, il importe que les particules **PV** soient suffisamment petites pour passer, en suspension dans un liquide, au travers de filtres dont le seuil de coupure est inférieur ou égal à 0,2 µm, sans les colmater. De telles facilité et efficacité de stérilisation par filtration sont particulièrement appréciées pour des médicaments injectables.

Concernant l'aptitude à l'injection des PV, il convient, pour une dose donnée de PA, de pouvoir injecter de faibles volumes de suspension liquide, et que cette suspension soit peu visqueuse. Il s'agit de pouvoir réduire les quantités d'excipient (**PV**) par rapport à la dose thérapeutique visée en **PA** et de fournir des **PV** ayant une taille la plus réduite possible, tout en augmentant la capacité de chargement du **PA.**

S'agissant de la spécification de biodégradabilité des **PV**, elle est d'autant meilleure que la taille des **PV** est réduite et permet leur élimination rapide.

En outre, il est appréciable de pouvoir réduire les quantités d'excipient (**PV**) pour des raisons économiques et pour améliorer la tolérance du médicament injectable.

### EXPOSE SUCCINCT DE L'INVENTION

Dans cet état de fait, un objectif essentiel est de pouvoir fournir de nouvelles **PV** qui forment spontanément, et sans l'aide de tensioactifs ou de solvants organiques, des suspensions aqueuses stables de **PV**.

Un autre objectif essentiel de la présente invention est de fournir de nouvelles **PV** en suspension aqueuse colloïdale stable ou sous forme pulvérulente et à base de poly(aminoacides) (PAA), ces nouvelles **PV** se devant de satisfaire au mieux aux spécifications 1 à 8 du cahier des charges susvisé.

Un autre objectif essentiel de l'invention est de perfectionner les particules divulguées dans la demande PCT WO 96/29991.

Un autre objectif essentiel de l'invention est de fournir une suspension nouvelle de **PV** dont on maîtrise parfaitement les caractéristiques, notamment en termes du taux de chargement en **PA** et en termes de contrôle de cinétique de libération du **PA.**

Un autre objectif essentiel de l'invention est de fournir des suspensions médicamenteuses injectables. Les spécifications, requises pour de telles suspensions, sont un faible volume d'injection et une faible viscosité. Il importe que la masse de particules colloïdales par dose d'injection soit le plus faible possible et ce sans limiter la quantité du principe actif **PA** transporté par ces particules, afin de ne pas nuire à l'efficacité thérapeutique.

Un autre objectif essentiel de l'invention est de fournir une suspension colloïdale aqueuse ou un solide pulvérulent comprenant des particules de vectorisation de principes actifs satisfaisant aux spécifications visées ci-dessus et qui constitue une forme galénique appropriée et convenable pour une administration, par exemple orale, à l'homme ou l'animal.

Un autre objectif essentiel de l'invention est de fournir une suspension colloïdale comprenant des particules de vectorisation de principes actifs filtrable sur des filtres de 0,2 µm à des fins de stérilisation.

Un autre objectif essentiel de l'invention est de proposer un procédé de préparation de particules (sèches ou en suspension dans un liquide) de PAA utiles, notamment, comme vecteurs de principes actifs, ledit procédé se devant d'être, plus simple à mettre en oeuvre, non dénaturant pour les principes actifs et devant en outre toujours permettre une maîtrise fine de la granulométrie moyenne des particules obtenues.

Un autre objectif essentiel de l'invention est l'utilisation des susdites particules en suspension aqueuse ou sous forme solide pour la préparation :
- de médicaments (e.g. vaccins), en particulier pour administration notamment orale, nasale, vaginale, oculaire, sous-cutanée, intraveineuse, intramusculaire, intradermique, intrapéritonéale, intracérébrale ou parentérale, les principes actifs de ces médicaments pouvant être, notamment, des protéines, des glycoprotéines, des peptides, des polysaccharides, des lipopolysaccharides, des oligonucléotides et des polynucléotides ;
- et/ou de nutriments,
- et/ou de produits cosmétiques ou phytosanitaires,
- et/ou de molécules organiques médicamenteuses.

Un autre objectif essentiel de la présente invention est de fournir des suspensions de **PV** submicroniques à base de PAA et susceptibles de servir de vecteur d'un **PA,** en particulier médicamenteux pour l'administration dudit **PA** à un organisme humain ou animal, ou bien encore d'un **PA** nutritionnel, phytosanitaire ou cosmétique.

Un autre objectif de la présente invention est de fournir un médicament, du type système à libération prolongée de principes actifs, qui soit aisé et économique à produire et qui soit, en outre, biocompatible et apte à assurer un très haut niveau de biodisponibilité du **PA.**

Un autre objectif essentiel de l'invention est de fournir un système de vectorisation de vaccin, qui soit non-immunogène intrinsèquement et en combinaison avec un ou plusieurs antigènes.

Les objectifs relatifs aux produits (parmi d'autres) sont atteints par la présente invention qui concerne, tout d'abord, une suspension colloïdale stable de particules structurées submicroniques susceptibles d'être utilisées, notamment pour la vectorisation de principe(s) actif(s) **PA,** ces particules étant des arrangements supramoléculaires individualisés (discrets) :
○ à base de polyaminoacides (PAA) amphiphiles, linéaires, à enchaînements peptidiques et comprenant au moins deux types différents d'aminoacides récurrents AAI hydrophiles et AAO neutres hydrophobes, les aminoacides de chaque type étant identiques ou différents entre eux,
○ et aptes à associer en suspension colloïdale à l'état non dissous, au moins un **PA** et à libérer celui-ci, notamment in vivo, de manière prolongée et/ou retardée, caractérisée :
   - en ce que le ou les AAI des chaînes polymères est (sont) choisi(s) parmi les aminoacides à chaîne latérale ionisable, les aminoacides naturels Glu et Asp sous forme carboxylique et/ou sous forme de sels étant particulièrement préférés,
   - en ce que le ou les AAO des chaînes polymères est(sont) choisi(s) dans le groupe comprenant les aminoacides neutres naturels, de préférence ceux appartenant au sous-groupe comportant : Leu, Ile, Val, Ala, Gly, Phe ;
   - en ce que les particules sont stables en phase aqueuse à pH compris entre 4 et 13 en l'absence de tensioactif(s),
   - par un taux de chargement Ta des particules de vectorisation avec l'insuline, exprimé en % de masse d'insuline associée par rapport à la masse et mesuré selon un mode opératoire Ma, Ta étant tel que :
      7 ≤ Ta
      de préférence, 8 < Ta ≤ 50
      et, plus préférentiellement encore, 10 ≤Ta ≤30
   - et par un diamètre hydrodynamique moyen Dh exprimé en nanomètres (nm) et mesuré selon un mode opératoire Md, Dh étant tel que :
      10 nm ≤ Dh ≤ 150 nm
      de préférence, 20 nm ≤ Dh ≤ 100 nm.

### EXPOSE DETAILLE DE L'INVENTION

Les modes opératoires Md et Ma pour les mesures Dh et Ta sont détaillés ci-après.

### Mode opératoire Md :

La poudre pulvérulente de PAA est mise en suspension dans une solution aqueuse de chlorure de sodium 0,15 M à pH 7,4, 25 °C et à une concentration en polymère comprise entre 0,01 et 0,5 g/l et, de préférence, égale à 0,1 g/l. Cette suspension est agitée 4 heures, puis introduite dans la cellule de diffusion d'un appareil de diffusion de la lumière, de type Brookhaven, fonctionnant avec un faisceau laser de longueur d'onde 488 nm et polarisé verticalement. Le diamètre hydrodynamique est calculé à partir de la fonction d'autocorrélation du champ électrique par le méthode des cumulants, comme décrit dans l'ouvrage «Surfactant Science Series » volume 22, Surfactant Solutions, Ed. R. Zana, chap. 3, M. Dekker, 1984.

### Mode opératoire Ma :

(a) Préparation d'une solution aqueuse d'insuline : De l'insuline recombinante humaine lyophilisée (Sigma n° 10259) est versée dans une solution HCl 0,1 N durant 5 min à 25°C. Cette solution est ensuite versée dans une solution de tampon phosphate qui est finalement neutralisée par ajout de NaOH de 0,1 N. La solution est laissée ensuite au repos 30 min à température ambiante, puis filtrée sur membrane acrodisc 0,8-0,2 µ. La masse d'insuline est calculée en fonction du volume souhaité de solution, afin d'obtenir une concentration de 60 UI/ml.
(b) Dispersion des particules de vectorisation en PAA à associer dans la solution d'insuline : Les **PV** lyophilisés sont ajoutés à la solution d'insuline, à raison de 10 mg **PV**/ml de solution. Ce mélange est agité au vortex à deux ou trois reprises, puis placée dans un agitateur à basculement à température ambiante pendant 18 heures. La suspension colloïdale est ensuite conservée à 4°C.
(c) Séparation de l'insuline libre de l'insuline associée et dosage de l'insuline libre : La solution, contenant l'insuline et les **PV** est centrifugée 1 heure sous 60 000 g à 20°C. Le surnageant est disposé dans des tubes munis d'une membrane d'ultrafiltration (seuil de coupure 100 000Da) et centrifugé sous 3 000 g, 2 heures à 20°C. L'insuline dans le filtrat est dosée par CLHP.

L'un des fondements inventifs de ces nouvelles particules de vectorisation PV, en suspension aqueuse colloïdale stable ou à l'état de solide pulvérulent, tient à la sélection originale d'un groupe de polymères et d'un méthodologie originale permettant d'obtenir des particules de taille submicronique, qui forment une suspension colloïdale aqueuse stable en l'absence de tensioactifs ou de solvants.

Un autre fondement inventif de ces nouvelles particules de vectorisation PV, en suspension aqueuse colloïdale stable ou à l'état de solide pulvérulent, tient à la sélection originale d'un groupe de particules structurées submicroniques particulières par leur taux de chargement Ta ≥ 7% et leur taille Dh ≤ 150 nm. Cette sélection est le fruit d'importantes et longues recherches sur le procédé d'obtention des particules. En effet, la réduction de taille et l'augmentation de la capacité de chargement des particules de polyaminoacides n'étaient, à priori, pas évidentes. Ainsi, en mettant en oeuvre le procédé d'obtention de nanoparticules de polyaminoacide enseigné dans la demande PCT WO 96/29991, l'homme de l'art n'a pas pu obtenir, « sur mesure », des particules qui répondent au nouveau cahier des charges, tel que défini supra.

Finalement, c'est en jouant sur les compositions des polymères et sur les conditions opératoires que les inventeurs ont pu isoler ces particules structurées de très petite taille qui sont à la base de PAA et qui présentent, de manière tout à fait surprenante et inattendue, une capacité de chargement Ta à l'insuline qui peut être jusqu'à trois fois supérieure à celle propre aux particules selon le WO 96/29991.

Avantageusement, la suspension selon l'invention est caractérisée en ce que les particules submicroniques ne tirent pas leur cohésion de la présence des trois composés suivants :
- I) huile
- II) phase aqueuse
- III) et au moins un copolyaminoacide linéaire non réticulé synthétique et comportant au moins deux types différents de comonomère aminoacide AAI hydrophile et AAO hydrophobe,
contrairement à la suspension de microparticules selon la demande de brevet FR 2 746 035.

La structure des polymères PAA et la nature des acides aminés, sont choisies de telle façon que :
- les chaînes de polymères se structurent spontanément sous forme de particules (**PV**) de petite taille,
- les particules forment une suspension colloïdale stable dans l'eau et en milieu physiologique,
- les **PV** s'associent avec des protéines ou autres **PA** en milieu aqueux, par un mécanisme spontané et non dénaturant pour la protéine,
- les **PV** libèrent les **PA** en milieu physiologique et, plus précisément, in vivo ; la cinétique de libération est fonction de la nature du polymère PAA précurseur des **PV.**

Ainsi, en jouant sur la structure particulière du PAA, on peut contrôler les phénomènes d'association et de libération du **PA** sur le plan cinétique et quantitatif.

Il est du mérite de la demanderesse d'avoir choisi, à titre de matériau constitutif des **PV,** une composition particulière de polyaminoacides qui sont amphiphiles et qui, donc, possèdent des propriétés des **PV** en PAA, à savoir :
- possibilité de former spontanément des suspensions colloïdales de **PV** compatibles avec le pH des milieux physiologiques rencontrés dans les applications thérapeutiques visées,
- association spontanée des **PA** avec des **PV** en l'absence d'autre agent que l'eau qui leur sert de solvant et qui, dans le cas des protéines, n'est pas dénaturant,
- possibilité de libérer le **PA** du complexe d'association **PA-PV,** dans des conditions physiologiques, avec des profils pharmacocinétique et pharmacodynamique, qui laissent présager des utilisations intéressantes dans le domaine thérapeutique (vectorisation **PA**),
et qui, par ailleurs, présentent de nouvelles propriétés qui sont :
- filtrabilité avec seuil de coupure inférieur ou égal à 0,2 µm à des fins de stérilisation,
- biodégradibilité améliorée,
- aptitude à l'injection optimisée.

Ces nouvelles propriétés ont pu être obtenues grâce aux fonctions techniques primaires des **PV** qui sont la petite taille nanométrique et le fort taux de chargement.

Pour définir un peu plus ces PAA, on peut indiquer qu'ils peuvent être du type ordonné, séquentiel alterné (blocs) ou du type désordonné, séquentiel aléatoire (statistique).

Ainsi, selon une première forme de réalisation des **PV** selon l'invention, les PAA constitutifs sont du type « bloc » et sont caractérisés par un rapport molaire AAO/(AAI+AAO) tel que :
- 10% ≤ AAO/(AAO + AAI) ≤ 70%,
- de préférence, 20% AAO/(AAI + AAO) ≤ 60%,
- et plus préférentiellement encore, 35 % ≤ AAO/(AAI + AAO) ≤ 50%.

Avantageusement, la longueur absolue de chaque bloc d'AAO, exprimé en nombre d'AAO est telle que :
- de préférence, AAO > 10,
- et, plus préférentiellement encore, 20 ≤ AAO ≤ 100.

Selon une deuxième forme de réalisation des **PV** selon l'invention, les PAA constitutifs sont du type « statistique » c'est-à-dire préparés par copolymérisation simultanée de monomères de AAI et AAO, et le rapport molaire AAO/(AAO + AAI) est tel que :
- AAO/(AAO + AAI) > 10 %,
- et, de préférence, AAO/(AAO + AAI) ≥ 20 %,
- et, plus préférentiellement encore, 30 % ≤ AAO/(AA1 + AAO) ≤ 70%.

Avantageusement, la masse molaire Mw de ces PAA statistiques est telle que :
- Mw ≥ 2 000 g/mol,
- de préférence, Mw ≥ 5 500 g/mol,
- et plus, préférentiellement encore, 5 500 g/mol ≤ Mw ≤ 200 000 g/mol.

Suivant une caractéristique préférée de l'invention, les PAA blocs ou statistiques constitutifs de particules ont des degrés de polymérisation DP compris entre 30 et 600, de préférence entre 50 et 200 et, plus préférentiellement encore, entre 60 et 150.

Avantageusement, les PAA constitutifs des particules PV sont des PAA « diblocs ».

La présente invention vise, non seulement des suspensions de particules nues, telles que définies ci-dessus, mais également des particules comprenant au moins un principe actif **PA** De préférence, la suspension selon l'invention est aqueuse et stable. Ces particules, chargées ou non en **PA,** sont, avantageusement, sous forme dispersée dans un liquide (suspension), de préférence aqueux, mais peuvent également être à l'état de solide pulvérulent, obtenu à partir de la suspension de **PV** telle que définie ci-dessus.

D'où il s'ensuit que l'invention concerne, outre une suspension colloïdale (de préférence aqueuse) de **PV,** un solide pulvérulent comprenant des **PV** et obtenu à partir de la suspension selon l'invention.

Un autre objet essentiel de l'invention se rapporte à la préparation des particules sélectionnées (telles que décrites ci-avant), aussi bien sous forme de suspension colloïdale que sous forme de solide pulvérulent .Le procédé de préparation considéré consiste, essentiellement, à synthétiser des PAA précurseur et à les transformer en particules structurées.

Plus précisément, il s'agit, tout d'abord, d'un procédé de préparation de particules structurées submicroniques susceptibles d'être utilisées, notamment pour la vectorisation de principe(s) actif(s), ces particules étant des arrangements supramoléculaires discrets :
- à base de polyaminoacides (PAA) amphiphiles linéaires, à enchaînements (-AAI hydrophiles et AAO hydrophobes, les aminoacides de chaque type étant identiques ou différents entre eux ;
- de diamètre moyen Dh, exprimé en nm et mesuré selon un mode opératoire Md, tel que :
   10 ≤ Dh ≤ 150
   de préférence, 20 ≤ Dh ≤ 100 ;
- d'une part aptes, à former une suspension colloïdale stable par un simple mélange dans un milieu aqueux, sans qu'il soit nécessaire d'y ajouter un solvant ni de tensioactifs ;
- et d'autre part, aptes à s'associer dans un milieu liquide, avec au moins un **PA** et, en particulier, avec l'insuline selon un taux de chargement Ta, exprimé en %, et mesuré selon un mode opératoire Ma tel que : 7 ≤ Ta, de préférence, 8 ≤ Ta ≤ 25, et, d'autre part, à libérer celui-ci, notamment in vivo, de manière prolongée et contrôlée.

Ce procédé est caractérisé en ce que :
1. on réalise une copolymérisation de monomères N-CarboxyAnhydrides d'aminoacides (NCA) d'au moins deux types différents, d'une part, des NCA-pAAI («pAAI» désignant un précurseur d'AAI) et, d'autre part, des NCA-AAO, en présence :
   ○ d'au moins un solvant polaire non aromatique, de préférence choisi dans le groupe comprenant : la N-MethylPyrrolidone (NMP), le DiMéthylFormamide (DMF), le DiméthylSulfoxyde (DMSO), le DiMéthylAcétamide (DMAc), la pyrrolidone, la NMP étant plus particulièrement préférée,
   ○ et, éventuellement d'au moins un co-solvant protique de préférence choisi dans le groupe comprenant la pyrrolidone, l'eau, les alcools ; le méthanol étant particulièrement préféré ;
2. on transforme les motifs récurrents pAAI du copolymère précurseur PAA des particules, en motifs récurrents AAI, en mettant en oeuvre une hydrolyse, de préférence acide, pour laquelle on ajoute au milieu organique susdécrit une phase aqueuse acide;
3. éventuellement on neutralise le milieu réactionnel ;
4. éventuellement on purifie le milieu réactionnel par dialyse pour obtenir une suspension aqueuse de particules structurées ;
5. éventuellement on concentre cette suspension ;
6. éventuellement on élimine le milieu liquide pour recueillir le solide pulvérulent comprenant les particules.

La première étape du procédé s'inspire des techniques connues de polymérisation d'anhydrides de N-carboxy-(-aminoacides (NCA), décrites, par exemple, dans l'article « Biopolymers, 15, 1869 (1976) » et dans l'ouvrage de H.R. KRICHELDORF «(-Aminoacid-N-carboxy Anhydride and Related Heterocycles » Springer Verlag (1987).

La mise en oeuvre de solvants de copolymérisation aprotiques non aromatiques polaire, judicieusement choisis, en évitant toute précipitation et le fait d'avoir recours à une hydrolyse acide en présence d'eau et de solvant organique polaire non aromatique, constituent des modalités nouvelles et inventives qui conduisent à des particules structurées, discrètes et submicroniques à forte capacité de chargement de PA, et qui forment une suspension colloïdale, stable en milieu aqueux. Ces particules ne sont nullement comparables à un précipité aggloméré macroscopique du genre de celui évoqué ci-avant à propos de la proposition antérieure (d).

Suivant une variante, à l'issue de l'étape 1, on précipite - de préférence dans l'eau - le copolymère poly(AOO)(pAAI) obtenu et on recueille ce précipité. Cette variante correspond à un mode discontinu de préparation de particules, dans lequel on isole le copolymère poly(AAO)(pAAI) sous forme de précipité formant un produit intermédiaire stable. Ce précipité peut être, par exemple, filtré, lavé et séché.

De manière plus préférée encore, les NCA-pAAI sont des NCA d'acide glutamique ou aspartique O-alkylé, par exemple des NCA-Glu-O-Me, NCA-Glu-O-Et ou NCA-Glu-O-Bz (Me = méthyle - Et = Ethyle - Bz = Benzyle).

De manière connue, la copolymérisation se déroule à une température comprise entre 20 et 120°C, à pression atmosphérique et en présence d'un initiateur aminé, e.g. : NH₃.

D'autres paramètres expérimentaux, comme la concentration en NCA et/ou polymère dans le solvant polaire non aromatique (de préférence le NMP), et/ou la concentration ou la nature du cosolvant protique, lors de la synthèse, seront ajustés selon les effets désirés et connus de l'homme de l'art.

L'hydrolyse acide (étape 2) est réalisée à l'aide d'eau et d'au moins un acide minéral, tel l'aide phosphorique ou chlorhydrique - ce dernier étant préféré - et/ou un acide organique, tel l'acide TriFluoroAcétique (TFA), l'acide acétique, l'acide dichloroacétique, ou les acides organosulfoniques.

Les proportions eau/acide -exprimées en parties en poids- dans une phase aqueuse acide d'hydrolyse sont, avantageusement :
- de 60/1 à 2/1,
- de préférence 40/1 à 2/1,
- et, plus préférentiellement encore, 20/1 à 2/1.

Les proportions phase aqueuse acide d'hydrolyse/NMP - exprimées en parties en poids- sont, avantageusement :
- de 5/100 à 200/100
- de préférence, 10/100 à 100/100
- et, plus préférentiellement encore, de 20/100 à 80/100.

D'autres paramètres, comme la concentration en polymère, la température du mélange réactionnel, le mode d'ajout de la phase aqueuse acide d'hydrolyse, l'emploi de pression réduite, la durée de la réaction, etc... ; sont ajustés selon les effets désirés et bien connus de l'homme de l'art.

La neutralisation (étape 3) s'opère, en pratique, par exemple à l'aide de soude.

On élimine ensuite le sel formé à l'issue de la neutralisation, ainsi que le solvant, par tout traitement de séparation physique approprié, par exemple par diafiltration (dialyse) (étape 4), filtration, modification pH, chromatographie...

Cela conduit à une suspension aqueuse de particules structurées qui peut être concentrée, par exemple par distillation ou tout autre moyen physique convenable : ultrafiltration, centrifugation.

Pour séparer, à l'étape 6, les particules de leur milieu liquide de suspension, on élimine, éventuellement, la phase aqueuse, par exemple par séchage (e.g. à l'étuve), par lyophilisation ou tout autre moyen physique convenable : ultrafiltration, centrifugation. On récupère, à l'issue de cette étape 6, un solide pulvérulent, de couleur blanche.

Selon une variante, l'étape de concentration peut être réalisée par un traitement chimique, tel qu'un abaissement du pH, qui transforme en acide la partie hydrophile des monomères glutamates, ce qui les rend insolubles dans l'eau. Ces intermédiaires PAA acides peuvent être filtrés, lavés et séchés. Lesdits intermédiaires acides peuvent être neutralisés avec une base chimique dans une étape ultérieure afin d'obtenir une suspension de particules.

Il est à noter que la mise en oeuvre des étapes 1, 2, 3, 4 et éventuellement 5 du procédé ci-dessus correspondant à une préparation d'une suspension colloïdale de particules submicroniques et à fort taux de chargement avec les **PA.**

Lors de cette préparation de suspension colloïdale, les PAA amphiphiles poly(AAO)(AAI) de l'étape 2 sont placés dans un milieu aqueux dans lequel au moins une partie des AAI est soluble et au moins une partie des AAO est insoluble. Les PAA existent sous forme de nanoparticules dans ce milieu aqueux.

Une alternative pour préparer la suspension de **PV** selon l'invention consiste à mettre en présence le solide pulvérulent, tel que décrit ci-dessus et en tant que produit et par son procédé d'obtention, avec un milieu aqueux, non solvant des AAO.

Pour effectuer l'association d'un ou plusieurs **PA** aux particules, il est possible de mettre en oeuvre plusieurs méthodes conformément à l'invention. Des exemples, non limitatifs, de ces méthodes sont énumérés ci-après.

Selon une première méthode, on effectue l'association de **PA** aux particules par mise en présence d'une phase liquide (aqueuse ou non) contenant le **PA** avec la suspension colloïdale de particules.

Selon une deuxième méthode, on effectue l'association du **PA** aux particules par mise en présence d'un **PA** à l'état solide avec la suspension colloïdale de particules. Le **PA** solide peut être, par exemple, sous forme de lyophilisat, de précipité, de poudre ou autre.

Selon une troisième méthode, on met en présence le solide pulvérulent (PAA), tel que décrit supra en tant que produit et par ses caractéristiques d'obtention, avec une phase liquide (aqueuse ou non) contenant le **PA**.

Selon une quatrième méthode, on met en présence le solide pulvérulent, tel que décrit supra en tant que produit et par ses caractéristiques d'obtention, avec le **PA** sous forme solide. On disperse ensuite ce mélange de solides, dans une phase liquide, de préférence une solution aqueuse.

Dans toutes ces méthodes, le **PA** utilisé peut être sous forme pure ou préformulée.

Compte tenu de la taille nanométrique des particules, la suspension peut être filtrée sur des filtres de stérilisation, ce qui permet d'obtenir, aisément et à moindre coût, des liquides médicamenteux injectables stériles. Le fait de pouvoir, grâce à l'invention, contrôler la taille des particules et atteindre des valeurs de Dh entre 25 et 100 nm, est un atout important.

La présente invention vise, également, de nouveaux produits intermédiaires du procédé décrit ci-dessus, caractérisés en ce qu'ils sont constitués par des copolymères PAA précurseurs de particules.

### APPLICATION INDUSTRIELLE

Selon un autre de ses aspects, l'invention concerne une suspension et/ou un solide pulvérulent, tels que définis ci-dessus et/ou tels qu'obtenus par le procédé présenté supra, cette suspension et ce solide comprenant au moins un principe actif, choisi, de préférence, parmi :
- les vaccins,
- les protéines et/ou les peptides, parmi lesquels les plus préférentiellement retenus sont: les hémoglobines, les cytochromes, les albumines, les interférons, les antigènes, les anticorps, l'érythropoïétine, l'insuline, les hormones de croissance, les facteurs VIII et IX, les interleukines ou leurs mélanges, les facteurs stimulants de l'hématopoïèse,
- les polysaccharides, l'héparine étant plus particulièrement sélectionnée,
- les acides nucléiques et, préférablement, les oligonucléotides d'ARN et/ou d'ADN,
- des molécules non petido-protéiques appartenant à diverses classes de chimiothérapie anticancéreuses et, en particulier, les anthracyclines et les taxoïdes,
- et leurs mélanges.

L'invention vise, également, une suspension et/ou lc solide pulvérulent chargé(s) en **PA** nutritionnel, phytosanitaire ou cosmétique.

Enfin, l'invention concerne une spécialité pharmaceutique, nutritionnelle, phytosanitaire ou cosmétique, caractérisée en ce qu'elle comporte une suspension et/ou du solide pulvérulent chargé(s) en **PA** et tels que définis ci-dessus.

Selon un autre de ses objets, l'invention vise, également, l'utilisation de ces **PV** (en suspension ou sous forme solide) chargées en **PA**, pour la fabrication de médicaments du type systèmes à libération contrôlée de **PA**.

Dans le cas de médicaments, il peut s'agir, par exemple de ceux administrables, de préférence par voie orale, nasale, vaginale, oculaire, sous-cutanée, intraveineuse, intramusculaire, intradermique, intrapéritonéale, intracérébrale ou parentérale.

Les applications cosmétiques envisageables sont, par exemple, les compositions comprenant un **PA** associé aux **PV** selon l'invention et applicables par voie transdermique.

Les produits phytosanitaires concernés peuvent être, par exemple, des herbicides, des pesticides, des insecticides, des fongicides, etc...

Les exemples qui suivent permettront de mieux comprendre l'invention dans ses différents aspects produit/procédé/application. Ces exemples illustrent la préparation de particules de polyaminoacides chargés ou non en principes actifs, de même qu'ils présentent les caractéristiques de structure et les propriétés de ces particules.

### LEGENDES DES FIGURES

Fig. 1 : Nanoparticules correspondant à un copolymère bloc la : leucine 50/Glutamate 50 obtenues selon l'enseignement du brevet WO 96/29991.

Fig. 2: Nanoparticules obtenues avec le copolymère bloc selon la présente invention (exemple 2). On notera que la barre ne représente plus ici que 50 nm.

Fig 3 Evolution de la concentration en glucose (moyenne en % basal sur 4 chiens) après injection d'une formulation de **PV** chargée en insuline à raison de 2 Ul/kg.

Fig. 4 Evolution de la concentration en insuline sérique (moyenne sur 4 chiens) après injection d'une formulation de **PV** chargée en insuline à raison de 2 Ul/kg.

### EXEMPLES

### EXEMPLE 1 -Obtention, en suspension aqueuse colloïadale stable et sous forme solide pulvérulente, de particules de vectorisation, à partir d'un polyaminoacide bloc, le poly(Leu/Glu) 40/80 dibloc

Dans un réacteur de 1 litre thermostaté à 20°C, on introduit sous agitation 112,4 g de NCA-GluOMe (0,60 mole) et 449 g de N-Méthyl Pyrrolidine-one-2 (NMP). Après dissolution, on ajoute 21,38 g d'une solution d'ammoniac 0,34 M dans le dioxanne-1,4 (1,25% molaire/NCA). La polymérisation est suivie par mesure du dioxyde de carbone dégagé dans une cloche à gaz et vérifiée par disparition des bandes de vibration caractéristiques des NCA à 1860 et 1790 cm-1. Après 30 min, on introduit une solution de 47,17 g de NCA Leucine (0,30 mole) dans 631 g de NMP. Après 10 min de réaction, la température est augmentée à 60°C. La polymérisation est suivie comme précédemment et est complète après 2 heures. La température du mélange réactionnel obtenu est augmentée à 80°C. A 350 g du mélange réactionnel obtenu en fin de l'étape 1 sont ajoutés 31,5 g d'acide chlorhydrique concentré aqueux (35%, 12M) sous agitation mécanique en 30 min. Le réacteur est alors mis sous pression réduite régulée à 600 mBar pendant 6 heures. Un mélange de 31,5 g d'acide chlorhydrique 35 % et de 126 g d'eau est alors ajouté sur 60 min, suivi d'une deuxième phase de vide à 250 mBar pendant 18 heures. Dans cet exemple, la ratio global Eau/Acide chlorhydrique pur est de 7,6/l en masse et le ratio phase aqueuse acide/NMP de 60/100 en masse.

Le mélange réactionnel est ensuite refroidi à 50°C, puis neutralisé par la soude aqueuse (35 % de la masse). La NMP et le chlorure de sodium formé lors de la neutralisation sont éliminés par diafiltration contre 20 volumes d'eau Milli Q, sur membrane de MWCO de 1000 Daltons (système Pellicon II, Millipore). On obtient ainsi une suspension colloïdale aqueuse stable de nanoparticules de vectorisation. La suspension de nanoparticules est finalement lyophilisée.

Les teneurs en motifs leucine sont déterminées par résonance magnétique nucléaire du proton (signaux à 2,10, 2,22 et 2,58 ppm our 4H du Glu et à 0,85 ppm pour 6H du Leu). La diamètre hydrodynamique moyen (Dh) est 70 nm (selon Md).

### EXEMPLE 2 - Association de l'insuline aux nanoparticules de poly(Leu/Glu) 40/80

On met en oeuvre le mode opératoire Ma. La concentration d'insuline libre, dosée par chromatographie CLHP, est égale à 0,59 mg/ml et on en déduit la concentration d'insuline associée égale à 1,51 mg/ml. La capacité de chargement d'une solution colloïdale de 10 mg/ml atteint 1,51 mg/ml d'insuline. Ainsi le rapport de la masse d'insuline associée à la masse bLE (Ta) est de 15,1 %.

### EXEMPLE 3- Obtention, en suspension aqueuse colloïdale stable et sous forme solide pulvérulente, de particules de vectorisation à partir d'un PAA bloc poly(Leu/glu) 25/70 dibloc.

146,4 g de NCA GluOMe sont dissous dans 586g de NMP auxquels sont ajoutés 18,43 g d'une solution d'ammoniac 0,48 M dans le méthanol. Lorsque la polymérisation des NCA GluOMe est complète, une solution de 43,9 g de NCA Leu dans 708 g de NMP est introduite et la polymérisation des NCA Leu est poursuivie jusqu'à disparition des monomères. Le milieu est alors porté à 80°C et on y ajoute, en goutte à goutte, durant 30 min à 1 heure, 129,4 g d'HCI 35 %. Un vide de 600 mBar est appliqué pendant 6 heures, puis 129,4 g d'HCI 35 % supplémentaires sont ajoutés en mélange avec 517,5 g d'eau. Un vide de 250 mBar est alors appliqué pendant 18 heures. Après cette étape, la température est réduite à 50°C, 1 litre d'eau est introduit, suivi de 280 ml de NaOH 35 % pour ramener le pH à 7,4. La suspension est ensuite filtrée (5 µm), dialysée (seuil de coupure 1 000 Da) dans de l'eau, pour éliminer le solvant et les sels, et enfin filtrée (0,22 µm). Cette suspension peut être utilisée directement ou subir des traitements ultérieurs, tels que la distillation de l'eau (étape 5) ou la lyophilisation (étape 6).

Le diamètre hydrodyamique moyen Dh (selon Md) est 14,8%. Le taux de chargement Ta de l'insuline, déterminé selon le mode opératoire Ma, est 35 nm.

### EXEMPLE 4- Obtention, en suspension colloïdale aqueuse stable, de nanoparticules de vectorisation, à partir d'un polyaminoacide bloc, le poly(Leu/Glu) 50/70 dibloc et caractéristiques des nanoparticules

Dans un réacteur de 0,5 litre thermostaté à 30°C, on introduit sous agitation 38,9 g de NCA-GluOMe (0,208 mole) et 156 g e N-Méthyl Pyrrolidine-one-2 (NMP). Après dissolution, on ajoute 5,79 g d'une solution d'ammoniac 0,407 M dans le méthanol (1,25 % molaire/NCA). La polymérisation est suivie par mesure du dioxyde de carbone dégagé dans une cloche à gaz et vérifiée par disparition des bandes de vibration caractéristiques des NCA à 1860 et 1790 cm-1. Après 30 min, on introduit une solution de 23,3 g de NCA Leucine (0;148 mole) dans 263 g de NMP. Après 10 min de réaction, la température est augmentée à 60°C. La polymérisation est suivie comme précédemment et est complète après 1-2 heures. La température du mélange réactionnel obtenu précédemment est augmentée à 80°C. 41.9 g d'acide chlorhydrique aqueux (35 % de la masse) sont ajoutés au milieu réactionnel sous agitation mécanique en 30 min. Le réacteur est alors mis sous pression réduite régulée à 600 mBar pendant 6 heures. Un mélange de 41.9 g d'acide chlorhydrique 35 % et de 167.5 g d'eau est alors ajouté sur 60 min, suivi d'une deuxième phase de vide à 250 mBar pendant 18 heures. Le mélange réactionnel est ensuite refroidi à 50°C, puis neutralisé par la soude aqueuse (35 % de la masse). La NMP et le chlorure de sodium formés lors de la neutralisation, sont éliminés par diafiltration contre 20 volumes d'eau Milli Q, sur membrane de MWCO de 1 000 Daltons (système Pellicon II, Millipore). On obtient ainsi une suspension colloïdale aqueuse stable de nanoparticules de vectorisation. La suspension de nanoparticules est finalement lyophilisée.

Le diamètre hydrodynamique moyen Dh est mesuré selon Md sur des suspensions aqueuses des lyophilisais. Le taux de chargement Ta de l'insuline est déterminé selon le mode opératoire Ma.

### EXEMPLE 5 Obtention, en suspension colloïdale aqueuse stable de nanoparticules de vectorisation, à partir d'un polyaminoacide bloc, le poly(Leu/Glu) 25/35 dibloc et caractéristiques des nanoparticules

Dans un réacteur de 0.5 litre thermostaté à 30°C, on introduit sous agitation 38,9 g de NCA-GluOMe (0,208 mole) et 156 g de N-Méthyl Pyrrolidine-one-2 (NMP). Après dissolution, on ajoute 5,78 g d'une solution d'ammoniac 0,452 M dans le méthanol (1,25 % molaire/NCA). La polymérisation est suivie par mesure de dioxyde de carbone dégagé dans une cloche à gaz et vérifiée par disparition des bandes de vibration caractéristiques des NCA à 1860 et 1790 cm-1. Après 30 min, on introduit une solution de 23,3 g de NCA Leucine (0,149 mole) dans 5 219 g de NMP. Après 10 min de réaction, la température est augmentée à 60°C. La polymérisation est suivie comme précédemment. Elle est complète après 1-2 heures. La température du mélange réactionnel obtenu précédemment est augmentée à 80°C. 42,0 g d'acide chlorhydrique aqueux (35 % de la masse) sont ajoutés au milieu réactionnel sous agitation mécanique en 30 min. Le réacteur est alors mis sous pression réduite régulée à 600 mBar pendant 6 heures. Un mélange de 42,0 g d'acide chlorhydrique 35 % et de 167,9 g d'eau est alors ajouté sur 60 min, suivi d'une deuxième phase de vide à 250 Mbar pendant 18 heures. Le mélange réactionnel est ensuite refroidi à 50°C, puis neutralisé par la soude aqueuse (35% de la masse). La NMP et le chlorure de sodium formés lors de la neutralisation, sont éliminés par diafiltration contre 20 volumes d'eau Milli Q, sur membrane de MWCO de 1000 Daltons (système Pellicon II, Millipore). On obtient une suspension colloïdale aqueuse stable de nanoparticules de vectorisation La suspension de nanoparticules est finalement lyophilisée.

Les teneurs en motifs leucine sont déterminées par résonance magnétique nucléaire du proton (signaux à 2,10, 2,22 et 2,58 ppm pour 4H du Glu et à 0,85 ppm pour 6H du Leu). Le diamètre hydrodynamique moyen Dh est mesuré selon Md sur des suspensions aqueuses des lyophilisats. Le taux de chargement de l'insuline est déterminé selon Ma.

### EXEMPLE 6 Obtention, en suspension colloïdale aqueuse stable de nanoparticules de vectorisation, d'un polyaminoacide bloc, le poly(Leu/glu) 50/150 dibloc et caractéristiques des nanophases

Dans un réacteur de 0,5 litre thermostaté à 30°C, on introduit sous agitation 46,4g de NCA-GluOMe (0,248 mole) et 186 g de N-Méthyl Pyrrolidine-one-2 (NMP). Après dissolution, on ajoute 6,90g d'une solution d'ammoniac 0,19 M dans le méthanol (1,25 % molaire/NCA). La polymérisation est suivie par mesure du dioxyde de carbone dégagé dans une cloche à gaz et vérifiée par disparition des bandes de vibration caractéristiques des NCA de 1 860 et 1 790 cm-1. Après 30 min, on introduit une solution de 12,97g de NCA Leucine (0,083 mole) dans 218 g de NMP. Après 10 min de réaction, la température est augmentée à 60°C.La polymérisation est suivie comme précédemment. Elle est complète après 1-2 heures. La température du mélange réactionnel obtenu précédemment est augmentée à 80°C. 40,3 g d'acide chlorhydrique aqueux (35 % de la masse) sont ajoutés au milieu réactionnel sous agitation mécanique en 30 min. Le réacteur est alors mis sous pression réduite régulée à 600 mBar pendant 6 heures. Un mélange de 40,3 g d'acide chlorhydrique 35 % et de 161,3 g d'eau est alors ajouté sur 60 min, suivi d'une deuxième phase de vide à 250 mBar pendant 18 heures. Le mélange réactionnel est ensuite refroidi à 50°C, puis neutralisé par la soude aqueuse (35 % de la masse).

La NMP et le chlorure de sodium formé lors de la neutralisation, sont éliminés par diafiltration contre 20 volumes d'eau Milli Q, sur membrane de NWCO de 1 000 Daltons (système Pellicon II, Millipore). On obtient une suspension colloïdale aqueuse stable de nanoparticules de vectorisation. La suspension de nanophases est finalement lyophilisée.

Les teneurs en motifs leucine sont déterminées par résonance magnétique nucléaire du proton (signaux à 2,10 ; 2,22 et 2,58 ppm pour 4H du Glu et à 0,85 ppm pour 6H du Leu). Le diamètre hydrodynamique moyen Dh est mesuré selon Md. Le taux de chargement de l'inuline est déterminé selon Ma.

### EXEMPLE 7 Exemple comparatif de la nature des particules formées avec l'enseignement du brevet PCT WO 96/29991

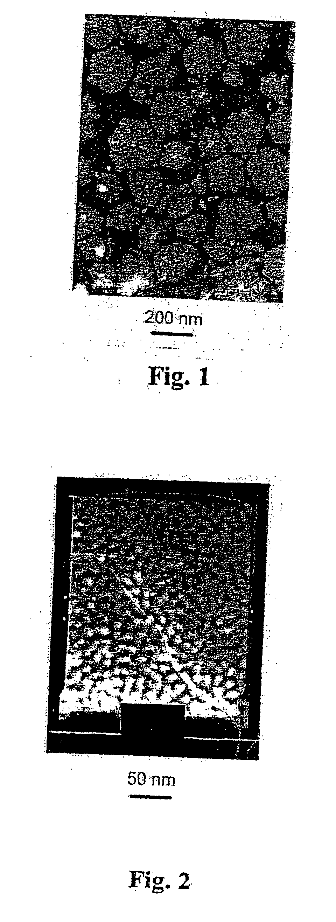

Les particules obtenues par l'enseignement du brevet WO 96/29991 sont celles apparaissant sur la Fig. 1. Avantageusement, les particules selon l'invention sont celles apparaissant sur la Fig. 2 annexée correspondant à une photographie prise au microscope électronique à transmission.

Les différences de morphologie et de taille apparaissent de manière flagrante en comparant la Fig. 1 qui représente des **PV** selon l'art antérieur, d'une part, et la Fig. 2 montrant des PV selon l'invention, d'autre part. On constate ici une différence notable de morphologie. Les PV de la Fig. 2 sont telles que la majorité des particules plus grande taille présente une forme allongée.

### EXEMPLE 8- Test de Stabilité d'une suspension colloïdale préparée selon l'exemple 2 avec le polymère poly(Leu/Glu) 40/80

La poudre pulvérulent de l'exemple 2 est dissoute à raison de 60 mg/ml de poudre dans un tampon phosphate. Le pH a été ajusté à 7.3 et l'osmolalité de la suspension a été ajustée à 300 mOsm/kg à l'aide d'une solution de NaCl 5M. La solution a été filtrée (0.22µm) avant d'être réparties à raison de 5ml en flacons stériles de 10ml. La stabilité des échantillons a été évaluée sur une durée de 4 mois. La moitié des échantillons a été gardée à 4°C (± 2°C) pendant que les autres échantillons sont maintenus à la température du laboratoire : 25°C (± 5°C). A des temps déterminés les échantillons sont prélevés du lieu de stockage et équilibrés 1 heure à température ambiante avant l'analyse. Les méthodes analytiques sont détailles, les résultats étant présentés sous forme de deux tableaux.
1) Vérification de l'homogénéité de la solution colloïdale: Sans agiter la suspension, on effectue trois prélèvements de 100 µl de façon à représenter l'état de la solution en haut, au milieu et en bas du flacon. L'indice de réfraction de chaque prélèvement est mesuré à 25°C sur un réfractomètre d'Abbe étalonné par rapport à l'eau pure. Trois lectures sont réalisés pour chaque prélèvement et l'on compare les trois moyennes. Toute variation de concentration de la solution se traduirait par une différence de l'indice de réfraction.
2) Mesure du diamètre hydrodynamique: Un prélèvement de 100 µl de la solution à analyser est dilué 120 fois par une solution de NaCl 0.15M et le Dh des particules colloïdales est mesuré selon le protocole Md.
3) Mesure de la viscosité: Les mesures sont réalisées sur des prélèvements de 0.75 ml à l'aide d'un rhéomètre AR1000 (TA instruments) équipé d'une géométrie Cône/Plan (cône 4 cm/2°C) à une température de 20.0°C +/-0.1°C (régulation par effet Pelletier). La courbe viscosité en fonction du gradient de cisaillement est enregistrée pour des gradients variant de 1 à 100 s⁻¹. A ces concentrations les solutions sont légèrement rhéofluidifiantes et la valeur de viscosité retenue est prise pour un gradient de 10 s⁻¹.

Les résultats obtenus après vieillissement à 4°C et 25°C sont rassemblés dans les tableau I et II.

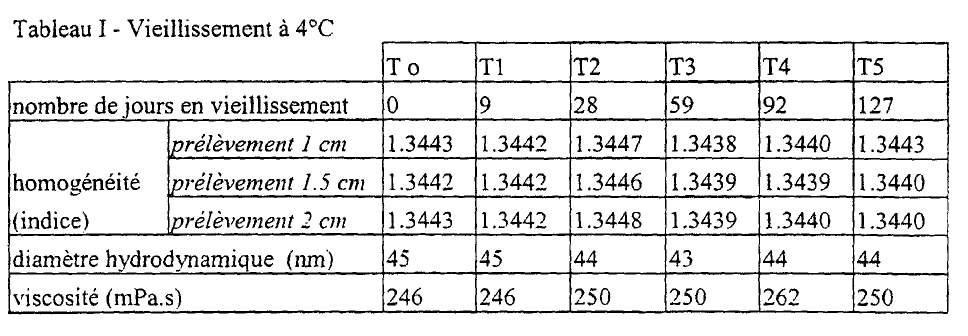

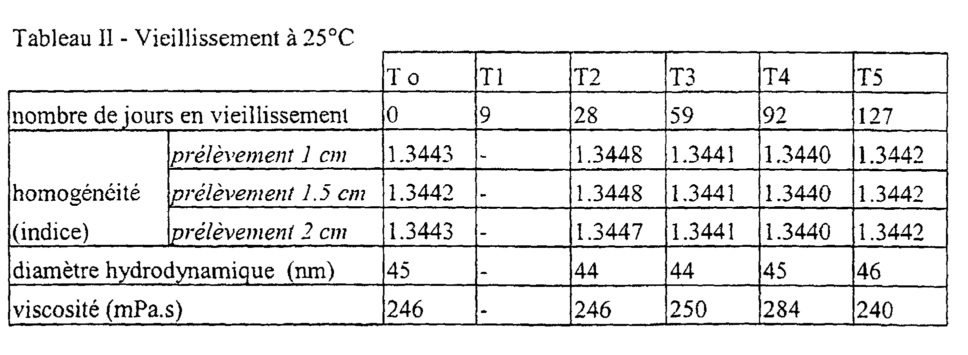

### EXEMPLE 9- Test de Libération d'insuline chez l'animal après administration d'une Suspension de Particules contenant de l'insuline

Une formulation est préparer à partir de **PV** (de l'exemple 3) et d'insuline, les quantité de chaque étant déterminées d'après les mesures de taux d'association (Ma).

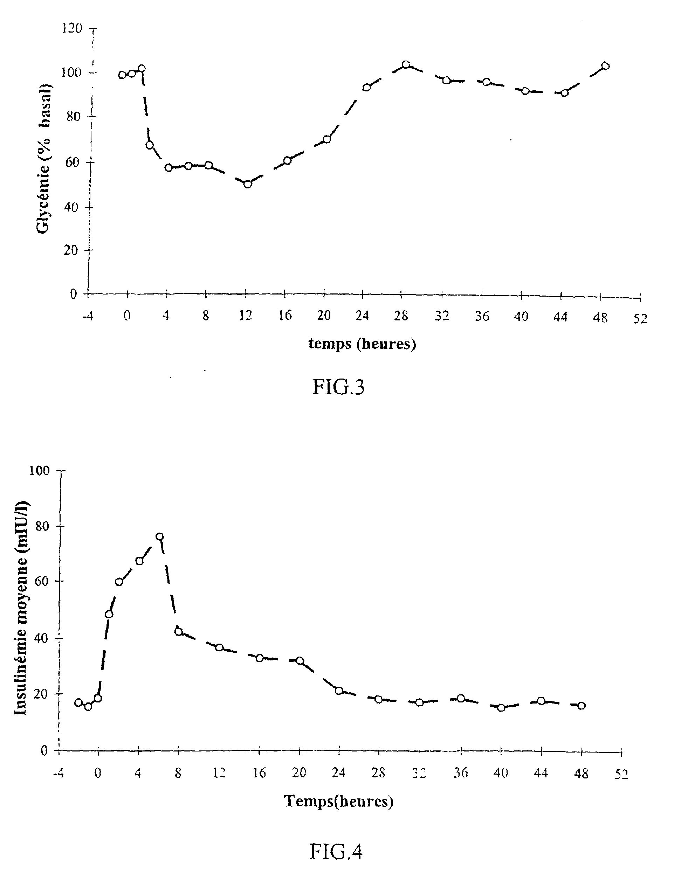

Une groupe de 4 chiens beagle (mâles et femelles) pesant entre 10 et 12 kg sont mis à jeun 18 heures. Une préparation est formulée et se compose de 80 Ul d'insuline t 56 mg de **PV** dans 1 ml de tampon PBS. Les chiens reçoivent ensuite une administration sous-cutanée de cette préparation d'insuline à raison de 2 Ul/kg de poids. Le sang prélevé pour un dosage du glucose et d'insuline avant (-2h, -1h et 0h) et après (1h, 2h, 4h, 6h, 12h, 16h, 20h, 24h, 28h, 32h, 36h, 40h, 44h, 48h) l'injection. Les concentrations en glucose sont mesurées dans les prélèvements par la méthode glucose-oxydasc et l'insuline sérique est dosée en utilisant une méthode radio-immunologique. La Fig. 3 donne la moyenne de l'évolution du glucose pour cette formulation. La fig. 4 donne la moyenne de l'évolution de l'insuline sérique pour cette formulation.

Cet exemple montre, au travers de l'activité biologique, la non dénaturation de la protéine ainsi que la possibilité de prolonger la libération de > 24h, deux aspects avantageux de la présente invention.

## Revendications

1. Suspension colloïdale de particules submicroniques susceptibles d'être utilisées, notamment pour la vectorisation de principe(s) actif(s) (PA), ces particules étant des arrangements supramoléculaires individualisés :
• à base de polyaminoacides (PAA) amphiphiles, linéaires, à enchaînements α-peptidiques et comprenant au moins deux types différents d'aminoacides récurrents AAI hydrophiles et AAO neutres hydrophobes, les aminoacides de chaque type étant identiques ou différents entre eux,
• et aptes à s'associer en suspension colloïdale à l'état non dissous, au moins un PA et à libérer celui-ci, notamment in vivo, de manière prolongée et/ou retardée,
**caractérisée :**
○ **en ce que** le ou les AAI est(sont) choisi(s) parmi des aminoacides à chaîne latérale ionisable, les aminoacides naturels Glu et Asp sous forme carboxylique et/ou sous forme de sels étant particulièrement préférés,
○ **en ce que** le ou est AAO est(sont) choisi(s) dans le groupe comprenant les aminoacides neutres naturels, de préférence ceux appartenant au sous-groupe comportant : Leu, Ile, Val, Ala, Pro, Phe ;
○ **en ce qu'**elle est stable à pH compris entre 4 et 13 en l'absence de tensioactif(s),
○ par un taux de chargement Ta avec l'insuline, exprimé en % de masse d'insuline associée par rapport à la masse et mesuré selon un mode opératoire Ma, Ta étant tel que :
Δ 7 ≤ Ta,
Δ de préférence, 8 ≤ Ta ≤ 50,
Δ et, plus préférentiellement encore, 10 ≤ Ta ≤ 30,
○ et par un diamètre hydrodynamique moyen Dh exprimé en nm et mesuré selon un mode opératoire Md, Dh étant tel que :
Δ 10 nm ≤ Dh ≤ 150 nm,
Δ de préférence, 20 nm ≤ Dh ≤ 100 nm.

2. Suspension selon la revendication 1, **caractérisée en ce que** les particules submicroniques ne tirent pas leur cohésion de la présence des trois composés suivants :
- I) huile
- II) phase aqueuse
- III) et au moins un copolyaminoacide linéaire non réticulé synthétique et comportant au moins deux types différents de comonomère aminoacide AAI hydrophile et AAO hydrophobe.

3. Suspension selon la revendication 1 ou 2, **caractérisée en ce que** les PAA constitutifs des particules sont des PAA « blocs » pour lesquels le rapport molaire AAO(AAI+AAO), exprimé en %, est tel que :
Δ 10% ≤ AAO/(AAI+AAO) ≤ 70 %,
Δ de préférence, 20 % ≤ AAO/(AAI+AAO) ≤ 60 %,
et pour lesquels le degré de polymérisation DP de la chaîne est compris entre 30 et 600, de préférence entre 50 et 100 et, plus préférentiellement encore, entre 60 et 150.

4. Suspension selon l'une quelconque des revendications 1 à 3, **caractérisée en ce que** les PAA constitutifs des particules sont des PAA « diblocs ».

5. Suspension selon l'une quelconque des revendications 1 à 4, **caractérisée en ce qu'**elle est aqueuse et stable.

6. Suspension selon l'une quelconque des revendications 1 à 5, **caractérisée en ce que** les particules comprennent au moins un principe actif PA.

7. Solide pulvérulent, **caractérisé en ce qu'**il est obtenu à partir de la suspension selon l'une quelconque des revendications 1 à 6.

8. Procédé de préparation du solide pulvérulent selon la revendication 7, **caractérisée en ce que** :
1) on réalise une copolymérisation de monomères formés par des anhydrides de N-CarboxyAminoacides (NCA) d'au moins deux types différents, d'une part. des NCA-pAAI (« pAAI » désignant des précurseurs d'AAI) et d'autre part, des NCA-AAO, en présence :
- d'au moins un solvant polaire non aromatique, de préférence choisi dans le groupe comprenant : la N-MéthylPyrrolidone (NMP), le DiMéthylFormamide (DMF), le DiMéthylsulfOxyde (DMSO), le DiMéthylAcétamide (DMAc), la pyrrolidone ; la NMP étant plus particulièrement préférée ;
- et éventuellement d'au moins un co-solvant sélectionné parmi les solvants aprotiques (de préférence le dioxanne-1,4) et/ou les solvants protiques (de préférence la pyrrolidone) et/ou l'eau et/ou les alcools, le méthanol étant particulièrement préféré ;
2) On transforme les motifs récurrents pAAI du copolymère obtenu à l'étape 1 en motifs récurrents AAI en mettant en oeuvre une hydrolyse - de préférence acide - pour laquelle on met en présence le copolymère obtenu à l'étape 1 avec une phase aqueuse d'hydrolyse acide + eau ;
3) on neutralise le milieu réactionnel ;
4) éventuellement on dialyse le milieu réactionnel pour purifier la suspension aqueuse de particules structurées ;
5) éventuellement on concentre cette suspension de l'étape 4 ;
6) on élimine le milieu liquide pour recueillir le solide pulvérulent comprenant les particules.

9. Procédé selon la revendication 8, **caractérisé en ce que**, à l'issue de l'étape 1, on précipite - de préférence dans l'eau - le copolymère poly (AAO)(pAAI) obtenu et on recueille le précipité.

10. Procédé de préparation de la suspension selon l'une quelconque des revendications 1 à 6, **caractérisé en ce que** l'on met en présence d'un milieu aqueux non solvant des AAO, le solide pulvérulent selon la revendication 7 et/ou le solide pulvérulent obtenu par le procédé selon la revendication 8.

11. Procédé de préparation de la suspension selon l'une quelconque des revendications 1 à 6, **caractérisé en ce qu'**il comprend les étapes 1, 2, 3, 4 et éventuellement 5 du procédé selon la revendication 8.

12. Procédé de préparation de la suspension selon la revendication 6, **caractérisé en ce que** l'on effectue l'association de PA aux particules, par mise en présence d'une phase liquide contenant le PA avec la suspension colloïdale de particules.

13. Procédé de préparation de la suspension selon la revendication 6, **caractérisé en ce que** l'on effectue l'association du PA aux particules par mise en présence d'un PA à l'état solide avec la suspension colloïdale de particules.

14. Procédé de préparation de la suspension selon la revendication 6, **caractérisé en ce que** l'on met en présence le solide pulvérulent selon la revendication 7 et /ou le solide pulvérulent obtenu par le procédé selon la revendication 8, avec une phase liquide contenant le PA.

15. Procédé de préparation de la suspension selon la revendication 6, **caractérisé en ce que** l'on met en présence le solide pulvérulent selon la revendication 7 et/ou le solide pulvérulent obtenu par le procédé selon la revendication 8, avec le PA sous forme solide et **en ce que** l'on disperse ce mélange de solides dans une phase liquide, de préférence une solution aqueuse.

16. Produits intermédiaires du procédé selon la revendication 8 ou 9, **caractérisés en ce qu'**ils sont constitués par des copolymères PAA précurseurs de particules.

17. Suspension selon la revendication 6 et/ou obtenue par le procédé selon l'une quelconque des revendications 12 à 15 et/ou solide pulvérulent selon la revendication 7 comprenant un moins un principe actif choisi, de préférence, parmi :
○ les vaccins,
○ les protéines et ou les peptides, parmi lesquels les plus préférentiellement retenus sont : les hémoglobines, les cytochromes, les albumines, les interférons, les antigènes, les anticorps, l'érythropoïétine, l'insuline, les hormones de croissance, les facteurs VIII et IX, les interleukines ou leurs mélanges, les facteurs stimulants de l'hématopoïèse,
○ les polysaccharides, l'héparine étant plus particulièrement sélectionnée.
○ les acides nucléiques et . préférablement, les oligonucléotides d'ARN et/ou d'ADN,
○ des molécules non peptido-protéiques appartenant à diverses classes de chimiothérapie anti-cancéreuses et, en particulier, les anthracyclines et les taxoïdes,
○ et leurs mélanges.

18. Suspension selon la revendication 6 et/ou suspension obtenue par le procédé selon l'une quelconque des revendications 12 à 15, et/ou solide pulvérulent selon la revendication 7, comprenant au moins un principe actif nutritionnel, phytosanitaire ou cosmétique.

19. Spécialité pharmaceutique, nutritionnelle, phytosanitaire ou cosmétique, **caractérisée en ce qu'**elle comporte une suspension et/ou du solide pulvérulent selon la revendication 17 ou 18.

## Patentansprüche

1. Kolloidale Suspension submikroner Partikel, die einer Verwendung insbesondere für die Vektorisierung von Wirkstoff(en) (WS) zugänglich sind, wobei diese Partikel supramolekulare, individualisierte Anordnungen sind:
• auf Basis amphiphiler, linearer, alpha-peptidisch verketteter Polyaminosäuren (PAS) und zumindest zwei verschiedene Arten von wiederholt auftretenden hydrophilen AAI Aminosäuren und neutralen hydrophoben AAO Aminosäuren umfassend, wobei die Aminosäuren jeder Art identisch oder voneinander verschieden sind,
• und geeignet, sich in kolloidaler Suspension in nicht gelöstem Zustand mit zumindest einem WS zu assoziieren und diesen, insbesondere in vivo, in lang anhaltender und/oder verzögerter Weise freizusetzen, **gekennzeichnet:**
**dadurch, dass** die AAI aus Aminosäuren mit ionisierbarer Seitenkette ausgewählt ist/sind, wobei die natürlichen Aminosäuren Glu und Asp in Carboxy-Form und/oder in Salzform besonderes bevorzugt sind;
dadurch, dass die AAO aus der Gruppe ausgewählt ist/sind, welche neutrale natürliche Aminosäure umfasst, vorzugsweise solche, die zur Leu, Ile, Val, Ala, Pro, Phe umfassenden Untergruppe gehören;
dadurch, dass sie bei einem pH von 4 bis 13 in Abwesenheit von oberflächenaktivem Stoff(en) stabil ist;
durch eine Beladungsrate Ta mit Insulin, ausgedrückt in Masse-% angelagerten Insulins im Verhältnis zur Masse und gemessen gemäß einer Verfahrensweise Ma, wobei Ta:
- 7 ≤ Ta,
- vorzugsweise 8 ≤ Ta ≤ 50,
- und noch bevorzugtererweise 10 ≤ Ta ≤ 30, ist
und durch einen mittleren hydrodynamischen Durchmesser Dh, ausgedrückt in nm und gemessen gemäß einer Verfahrensweise Md, wobei Dh
- 10 nm ≤ Dh ≤ 150 nm,
- vorzugsweise 20 nm ≤ Dh ≤ 100 nm, ist.

2. Suspension gemäß Anspruch 1, **dadurch gekennzeichnet, dass** die submikronen Partikel ihren Zusammenhalt nicht aus den folgenden drei Verbindungen beziehen:
I) Öl
II) wässrige Phase
III) und zumindest eine lineare, nicht verzweigte synthetische Copolyaminosäure, die zumindest zwei verschiedene Arten von hydrophilen Aminosäurecomonomeren AAI und hydrophoben Aminosäurecomonomeren AAO umfasst.

3. Suspension gemäß Anspruch 1 oder 2, **dadurch gekennzeichnet, dass** die die Partikel bildenden PAS "Block"-PAS sind, für welche das molare Verhältnis AAO/(AAI+AAO), ausgedrückt in %,
- 10 % ≤ AAO/(AAI+AAO) ≤ 70 %,
- vorzugsweise 20 % ≤ AAO/(AAI+AAO) ≤ 60 % ist,
und für welche der Polymerisationsgrad DP der Kette zwischen 30 und 600, vorzugsweise zwischen 50 und 100, und noch bevorzugterweise zwischen 60 und 150 liegt.

4. Suspension gemäß einem der Ansprüche 1 bis 3, **dadurch gekennzeichnet, dass** die die Partikel bildenden PAS "Doppelblock"-PAS sind.

5. Suspension gemäß einem der Ansprüche 1 bis 4, **dadurch gekennzeichnet, dass** sie wässrig und stabil ist.

6. Suspension gemäß einem der Ansprüche 1 bis 5, **dadurch gekennzeichnet, dass** die Partikel zumindest einen WS umfassen.

7. Pulverförmiger Feststoff, **dadurch gekennzeichnet, dass** er aus der Suspension gemäß einem der Ansprüche 1 bis 6 erhalten ist.

8. Verfahren zur Herstellung pulverförmigen Feststoffs gemäß Anspruch 7, **gekennzeichnet durch**:
1) Herstellen eines Copolymerisats aus Monomeren, das aus Anhydriden von N-Carboxyaminosäuren (NCA) von zumindest zwei verschiedenen Arten gebildet wird, zum einen aus NCA-pAAI (wobei "pAAI" Vorläufer von AAI bezeichnet), und zum anderen aus NCA-AAO, in Anwesenheit
- zumindest eines polaren, nicht aromatischen Lösungsmittels, vorzugsweise ausgewählt aus der Gruppe, die umfasst: n-Methylpyrrolidon (NMP), Dimethylformamid (DMF), Dimethylsulfoxid (DMSO), Dimethylacetamid (DMAc), Pyrrolidon, wobei NMP besonders bevorzugt ist;
- und optional zumindest eines Co-Lösungsmittels, das ausgewählt ist aus den aprotischen Lösungsmitteln (vorzugsweise Dioxan-1,4) und/oder den protischen Lösungsmitteln (vorzugsweise Pyrrolidon) und/oder Wasser und/oder Alkoholen, wobei Methanol besonders bevorzugt ist;
2) Umwandeln der in Schritt 1 erhaltenen, wiederholt auftretenden Copolymer-pAAI-Muster, in wiederholt auftretende AAI-Muster unter Verwendung einer - vorzugsweise sauren- Hydrolyse, für welche das in Schritt 1 erhaltene Copolymer mit einer wässrigen Säure + Wasser-Hydrolysephase zusammengebracht wird;
3) Neutralisieren des Reaktionsansatzes;
4) Optional Dialysieren des Reaktionsansatzes zum Reinigen der wässrigen Suspension der strukturierten Partikel;
5) Optional Konzentrieren dieser Suspension aus Schritt 4;
6) Entziehen des wässrigen Milieus zum Gewinnen des die Partikel umfassenden pulverförmigen Feststoffs.

9. Verfahren gemäß Anspruch 8, **dadurch gekennzeichnet, dass** nach Abschluss von Schritt 1 das erhaltene Poly (AAO)(pAAI) Copolymer - vorzugsweise in Wasser - präzipitiert und das Präzipitat gewonnen wird.

10. Verfahren zur Herstellung der Suspension gemäß einem der Ansprüche 1 bis 6, **dadurch gekennzeichnet, dass** man ein nicht AAO lösendes wässriges Lösungsmittel, den pulverförmigen Feststoff gemäß Anspruch 7 und/oder den durch das Verfahren gemäß Anspruch 8 erhaltenen pulverförmigen Feststoff zusammenbringt.

11. Verfahren zur Herstellung der Suspension gemäß einem der Ansprüche 1 bis 6, **dadurch gekennzeichnet, dass** es die Schritte 1, 2, 3, 4, und optional 5 des Verfahrens gemäß Anspruch 8 umfasst.

12. Verfahren zur Herstellung der Suspension gemäß Anspruch 6, **dadurch gekennzeichnet, dass** man die Assoziierung des WS mit den Partikeln durch Zusammenbringen einer den WS enthaltenden Flüssigphase mit der kolloidalen Partikelsuspension bewirkt.

13. Verfahren zur Herstellung der Suspension gemäß Anspruch 6, **dadurch gekennzeichnet, dass** man die Assoziierung des WS mit den Partikeln durch Zusammenbringen eines WS im festen Zustand mit der kolloidalen Partikelsuspension bewirkt.

14. Verfahren zur Herstellung der Suspension gemäß Anspruch 6, **dadurch gekennzeichnet, dass** man den pulverförmigen Feststoff gemäß Anspruch 7 und/oder den durch das Verfahren gemäß Anspruch 8 erhaltenen pulverförmigen Festsstoff mit einer den WS enthaltenden Flüssigphase zusammenbringt.

15. Verfahren zur Herstellung der Suspension gemäß Anspruch 6, **dadurch gekennzeichnet, dass** man den pulverförmigen Feststoff gemäß Anspruch 7 und/oder den durch das Verfahren gemäß Anspruch 8 erhaltenen pulverförmigen Festsstoff mit dem WS in fester Form zusammenbringt, und dadurch, dass man dieses Gemisch von Feststoffen in einer Flüssigphase, vorzugsweise einer wässrigen Lösung, auflöst.

16. Zwischenprodukte des Verfahrens gemäß Anspruch 8 oder 9, **dadurch gekennzeichnet, dass** sie aus Partikelvorläufer-PAA-Copolymeren bestehen.

17. Suspension gemäß Anspruch 6 und/oder erhalten durch das Verfahren gemäß einem der Ansprüche 12 bis 15 und/oder pulverförmiger Feststoff gemäß Anspruch 7, umfassend zumindest einen Wirkstoff, der vorzugsweise ausgewählt ist aus:
- Vakzinen,
- Proteinen und/oder Peptiden, unter welchen die bevorzugtesten sind: Hämoglobine, Cytochrome, Albumine, Interferone, Antigene, Antikörper, Erythropoetin, Insulin, Wachstumshormone, Faktoren VIII und IX, Interleukine oder ihre Mischungen, Aktivierungsfaktoren der Hämatopoese,
- Polysaccharide, wobei insbesondere Heparin ausgewählt wird,
- Nukleinsäuren und vorzugsweise RNA und/oder DNA-Oligonukleotide,
- Nicht-Peptid-Protein-Moleküle, die zu verschiedenen Klassen von Anti-Krebs-Chemotherapie gehören und insbesondere Anthracycline und Taxoide,
- und ihre Mischungen.

18. Suspension gemäß Anspruch 6 und/oder erhalten durch das Verfahren gemäß einem der Ansprüche 12 bis 15 und/oder pulverförmiger Feststoff gemäß Anspruch 7, umfassend zumindest einen Ernährungs-, Pflanzenschutz- oder Kosmetik-Wirkstoff.

19. Pharmazeutik-, Ernährungs-, Pflanzenschutz- oder Kosmetikspezialität, **dadurch gekennzeichnet, dass** sie eine Suspension und/oder einen pulverförmigen Feststoff gemäß Anspruch 17 oder 18 umfasst.

## Claims

1. A colloidal suspension of submicronic particles which can be used, in particular for carrying active principle(s) **(PA(s)),** these particles being individualized supramolecular arrangements:
• based on linear, amphiphilic polyamino acids (PAA), with a-peptide linkages and comprising at least two different types of recurring amino acids: hydrophilic AAI and hydrophobic neutral AAO, the amino acids of each type being mutually identical or different,
• and capable of combining in colloidal suspension, in the nondissolved state, at least one **PA** and of releasing it, in particular in vivo, in a prolonged and/or delayed manner, **characterized:**
○ **in that** the AAI(s) is(are) chosen from amino acids with an ionizable side chain, the natural amino acids Glu and Asp in carboxylic form and/or in the form of salts being particularly preferred,
○ **in that** the or is AAO(s) is(are) chosen from the group comprising natural neutral amino acids, preferably those belonging to the subgroup comprising: Leu, Ile, Val, Ala, Gly, Phe;
○ **in that** it is stable at pH between 4 and 13 in the absence of surfactant(s),
○ by a load factor Ta with insulin, expressed as % of combined insulin mass relative to the mass and measured according to a procedure Ma, Ta being such that:
Δ 7 ≤ Ta,
Δ preferably, 8 ≤ Ta ≤ 50,
Δ and, still more preferably, 10 ≤ Ta ≤ 30,
○ and by a mean hydrodynamic diameter Dh expressed in nm and measured according to a procedure Md, Dh being such that:
Δ 10 nm ≤ Dh ≤ 150 nm,
Δ preferably, 20 nm ≤ Dh ≤ 100 nm.

2. The suspension as claimed in claim 1,
**characterized in that** the submicronic particles do not acquire their cohesion from the presence of the following three compounds:
- I) oil
- II) aqueous phase
- III) and at least one synthetic non-crosslinked linear copolyamino acid comprising at least two different types of amino acid comonomer: hydrophilic AAI and hydrophobic AAO.

3. The suspension as claimed in claim 1 or 2, **characterized in that** the constituent PAAs of the particles are "block" PAAs for which the AAO(AAI+AAO) molar ratio, expressed as %, is such that:
Δ 10%≤AAO/(AAI+AAO)≤70%,
Δ preferably, 20%≤ AAO/(AAI+AAO)≤60%,
and for which the degree of polymerization DP of the chain is between 30 and 600, preferably between 50 and 100, and still more preferably between 60 and 150.

4. The suspension as claimed in any one of claims 1 to 3, **characterized in that** the constituent PARs of the particles are "diblock" PARs.

5. The suspension as claimed in any one of claims 1 to 4, **characterized in that** it is aqueous and stable.

6. The suspension as claimed in any one of claims 1 to 5, **characterized in that** the particles comprise at least one active principle PA.

7. A pulverulent solid, **characterized in that** it is obtained from the suspension as claimed in any one of claims 1 to 6.

8. A method for preparing the pulverulent solid as claimed in claim 7, **characterized in that**:
1) copolymerization of monomers consisting of anhydrides of N-CarboxyAmino acids (NCA) of at least two different types, on the one hand, NCAs-pAAI ("pAAI" designating precursors of AAI) and, on the other hand, NCAs-AAO, is carried out in the presence:
- of at least one nonaromatic polar solvent, preferably chosen from the group comprising: N-MethylPyrrolidone (NMP), DiMethylFormamide (DMF), DiMethyl SulfOxide (DMSO), DiMethylAcetamide (DMAc), pyrrolidone; NMP being most particularly preferred;
- and optionally of at least one cosolvent selected from aprotic solvents (preferably 1,4-dioxane) and/or protic solvents (preferably pyrrolidone) and/or water and/or alcohols, methanol being particularly preferred;
2) the recurring pAAI motifs of the copolymer obtained in step 1 are converted to recurring AAI motifs, using hydrolysis, preferably acid hydrolysis, for which the copolymer obtained in step 1 is brought into contact with an aqueous phase for acid hydrolysis + water;
3) the reaction medium is neutralized;
4) optionally, the reaction medium is dialyzed in order to purify the aqueous suspension of structured particles;
5) optionally, this suspension of step 4 is concentrated;
6) the liquid medium is removed in order to collect the pulverulent solid comprising the particles.

9. The method as claimed in claim 8, **characterized in that**, at the end of step 1, the copolymer poly(AAO)(pAAI) obtained is precipitated - preferably in water - and the precipitate is recovered.

10. A method for preparing the suspension as claimed in any one of claims 1 to 6, **characterized in that** the pulverulent solid as claimed in claim 7 and/or the pulverulent solid obtained by the method as claimed in claim 8 are brought into contact with a nonsolvent aqueous medium for the AAOs.

11. A method for preparing the suspension as claimed in any one of claims 1 to 6, **characterized in that** it comprises steps 1, 2, 3, 4 and optionally 5 of the method as claimed in claim 8.

12. A method for preparing the suspension as claimed in claim 6, **characterized in that** the combination of PA with the particles is carried out by bringing a liquid phase containing the PA into contact with the colloidal suspension of particles.

13. A method for preparing the suspension as claimed in claim 6, **characterized in that** the combination of the PA with the particles is carried out by bringing a PA in the solid state into contact with the colloidal suspension of particles.

14. A method for preparing the suspension as claimed in claim 6, **characterized in that** the pulverulent solid as claimed in claim 7 and/or the pulverulent solid obtained by the method as claimed in claim 8 are brought into contact with a liquid phase containing the PA.

15. A method for preparing the suspension as claimed in claim 6, **characterized in that** the pulverulent solid as claimed in claim 7 and/or the pulverulent solid obtained by the method according to claim 8 are brought into contact with the PA in solid form and **in that** this mixture of solids is dispersed in a liquid phase, preferably an aqueous solution.

16. An intermediate product of the method as claimed in claim 8 or 9, **characterized in that** it consists of PAA copolymers which are precursors of particles.

17. The suspension as claimed in claim 6 and/or obtained by the method as claimed in any one of claims 12 to 15 and/or the pulverulent solid as claimed in claim 7 comprising at least one active principle preferably chosen from:
○ vaccines,
○ proteins and/or peptides, among which those most preferably selected are: hemoglobins, cytochromes, albumins, interferons, antigens, antibodies, erythropoietin, insulin, growth hormones, factors VIII and IX, interleukins or mixtures thereof, hematopoiesis-stimulating factors,
○ polysaccharides, heparin being more particularly selected,
○ nucleic acids and, preferably, RNA and/or DNA oligonucleotides,
○ non-petido-protein molecules belonging to various anticancer chemotherapy classes and, in particular, anthracyclines and taxoids,
○ and mixtures thereof.

18. The suspension as claimed in claim 6 and/or the suspension obtained by the method as claimed in any one of claims 12 to 15, and/or the pulverulent solid as claimed in claim 7, comprising at least one nutritional, plant-protection or cosmetic active principle.

19. A pharmaceutical, nutritional, plant-protection or cosmetic proprietary product, **characterized in that** it comprises a suspension and/or the pulverulent solid as claimed in claim 17 or 18.
